# EUROPÄISCHE PATENTSCHRIFT

(11) **EP 4 275 844 B1**
(45) Veröffentlichungstag und Bekanntmachung des Hinweises auf die Patenterteilung: **06.11.2024**
(21) Anmeldenummer: 23172709.0
(22) Anmeldetag: 11.05.2023
(51) Int. Cl.: B25F 5/00, A61F 11/14

(54) **SCHUTZSYSTEM FÜR EIN ELEKTROWERKZEUG**
PROTECTION SYSTEM FOR A POWER TOOL
SYSTÈME DE PROTECTION POUR OUTIL ÉLECTRIQUE

(30) Priorität: 13.05.2022 DE 202022102628 U
(43) Veröffentlichungstag der Anmeldung: 15.11.2023
(73) Patentinhaber: Einhell Germany AG, 94405 Landau / Isar (DE)
(72) Erfinder: MAYR, Stefan, 94405 Landau/Isar (DE)
(74) Vertreter: Hofstetter, Schurack & Partner

(56) Entgegenhaltungen:
- EP-A1- 3 610 989
- EP-A1- 3 902 296
- EP-B1- 3 445 065
- WO-A2-2016/203315
- US-A1- 2019 083 320

## Beschreibung

Die vorliegende Erfindung betrifft ein Schutzsystem für ein Elektrowerkzeug und ein Elektrowerkzeugsystem mit einem solchen Schutzsystem. Ein derartiges Schutzsystem ist zum Beispiel aus der US 2019/083320 A1 bekannt.

Bei Elektrowerkzeugen, insbesondere Elektrowerkzeugen zum Trennen, Schleifen oder Bohren, kann es zu einer starken Belastung oder Überlastung des elektrischen Verbrauchers des Elektrowerkzeugs, insbesondere eines Elektromotors des Elektrowerkzeugs, und/oder zu einer starken Belastung oder Überlastung der Elektronik kommen, wenn dem elektrischen Verbraucher durch das zu bearbeitende Werkstück ein sehr hoher Widerstand entgegengesetzt wird. Bei akkubetriebenen Elektrowerkzeugen kann eine solche Überlastung auch zur Beschädigung von Akkumulatorzellen führen. Dies kann insbesondere relevant sein, wenn ein Benutzer selbst für einen Vorschub des Elektrowerkzeugs bezüglich des Werkstücks sorgt oder jedenfalls teilweise ein solcher manueller Vorschub vorgesehen ist, wie etwa bei Handkreissägen, Winkelschleifern, Bohrmaschinen oder sonstigen Elektrowerkzeugen zum Trennen, Schleifen oder Bohren. Außerdem kann es möglich sein, insbesondere wenn das Elektrogerät mit einem Akkupack, beispielsweise Wechsel-Akkupack, betreibbar ist, dass eine maximale Stromabgabe des Akkupacks höher ist, als ein maximal für den elektrischen Verbraucher zulässiger oder tolerierbarer Strom, sodass der elektrische Verbraucher im Zweifelsfall beschädigt werden könnte.

Es ist bekannt, die Stromaufnahme und/oder Temperatur des elektrischen Verbrauchers eines Elektrowerkzeugs zu überwachen und das Elektrowerkzeug gegebenenfalls abzuschalten, wenn die Stromaufnahme und/oder Temperatur einen jeweiligen zulässigen Grenzwert überschreitet. Dies ist jedoch zum einen nachteilhaft hinsichtlich des Benutzerkomforts, da der Benutzer in seiner Arbeit unterbrochen wird. Darüber hinaus führt die Unterbrechung des Betriebs des Elektrowerkzeugs gegebenenfalls zu Qualitätseinbußen bei der Bearbeitung des Werkstücks, insbesondere wenn Schneiden zum Stillstand gebracht werden. Außerdem muss bei einem solchen Ansatz der zulässige Grenzwert für den Strom relativ konservativ ausgelegt werden. Ein Nachteil der temperaturabhängigen Abschaltung kann es sein, dass es relativ lange dauern kann, bis der elektrische Verbraucher ausreichend abgekühlt ist.

Es ist eine Aufgabe der vorliegenden Erfindung, eine Möglichkeit zum Schutz eines Elektrowerkzeugs oder seiner Komponenten vor Überlastung anzugeben, durch die ein zwangsweises Abschalten des Elektrowerkzeugs möglichst vermieden wird.

Diese Aufgabe wird gelöst durch den Gegenstand des unabhängigen Anspruchs. Vorteilhafte Weiterbildungen und bevorzugte Ausführungsformen sind Gegenstand der abhängigen Ansprüche.

Die Erfindung beruht auf dem Gedanken, ein Akkupack für das Elektrowerkzeug derart mit einem Gehörschutz für einen Benutzer des Elektrowerkzeugs zu koppeln, dass das Akkupack den Gehörschutz abhängig von einem Wert eines Betriebsparameters des Elektrowerkzeugs dazu veranlasst, über ein Audioausgabegerät eine akustische Warnmeldung zu erzeugen.

Gemäß einem Aspekt der Erfindung wird ein Schutzsystem für ein Elektrowerkzeug angegeben, das Schutzsystem aufweisend ein Akkupack, das mit dem Elektrowerkzeug zur Versorgung eines elektrischen Verbrauchers, insbesondere eines Elektromotors, des Elektrowerkzeugs mit elektrischer Energie verbindbar ist. Das Schutzsystem weist sowie einen, insbesondere separat zu dem Elektrowerkzeug und dem Akkupack vorgesehenen, Gehörschutz für einen Benutzer des Elektrowerkzeugs auf. Das Akkupack weist einen ersten Steuerschaltkreis auf, der Gehörschutz weist einen zweiten Steuerschaltkreis auf. Der erste Steuerschaltkreis ist dazu eingerichtet, eine Information betreffend einen Wert eines Betriebsparameters des Elektrowerkzeugs direkt oder indirekt an den zweiten Steuerschaltkreis des Gehörschutzes zu übermitteln. Der Gehörschutz weist ein Audioausgabegerät, beispielsweise einen oder mehrere Lautsprecher, auf und der zweite Steuerschaltkreis ist dazu eingerichtet, das Audioausgabegerät abhängig von der übermittelten Information dazu anzusteuern, eine akustische Warnausgabe zu erzeugen.

Der Betriebsparameter kann beispielsweise einem Betriebsstrom des elektrischen Verbrauchers entsprechen, beispielsweise einem Strom, den eine Energiequelle, insbesondere ein Akkupack, während des Betriebs des Elektrowerkzeugs an den elektrischen Verbraucher, insbesondere den Elektromotor, abgibt. Je nach Ausführung des Elektrowerkzeugs und des Verbrauchers können jedoch auch andere Betriebsparameter verwendet werden, wie etwa eine Betriebsspannung, beispielsweise eine Ausgangsspannung des Akkupacks, ein Ladezustand des Akkupacks, eine Temperatur, beispielsweise eine Temperatur einer elektronischen Komponente des Elektrowerkzeugs oder des elektrischen Verbrauchers, ein Fluidfluss eines Betriebsmittels, also insbesondere eine Fluidflussrate oder eine Fluidflussgeschwindigkeit, oder eine Kombination aus den genannten Größen, etwa ein Produkt aus Fluiddruck und Fluidflussgeschwindigkeit, eine elektrische Leistung oder dergleichen. Der Betriebsparameter kann auch eine abgeleitete Größe sein, etwa ein Drehmoment, das beispielsweise aus Signaturen abgeleitet werden kann, die im Betriebsstrom oder der Ausgangsspannung auftreten, sogenannte Ripples.

Die Kommunikation zwischen dem Akkupack, insbesondere dem ersten Steuerschaltkreis, und dem Gehörschutz, insbesondere dem zweiten Steuerschaltkreis, kann grundsätzlich drahtlos oder drahtgebunden erfolgen. Vorzugsweise erfolgt die Kommunikation drahtlos, beispielsweise über Funk. Insbesondere kann die Schutzvorrichtung eine mit dem ersten Steuerschaltkreis verbundene erste Kommunikationsschnittstelle für das Akkupack aufweisen und eine mit dem zweiten Steuerschaltkreis verbundene zweite Kommunikationsschnittstelle für den Gehörschutz.

Die Übermittlung der Information kann dann direkt oder indirekt von der ersten Kommunikationsschnittstelle an die zweite Kommunikationsschnittstelle erfolgen. Unter einer direkten Übermittlung kann dabei verstanden werden, dass mittels der ersten Kommunikationsschnittstelle ein Funksignal oder ein drahtgebundenes Signal erzeugt wird und direkt ohne Zwischenschaltung einer weiteren Stelle an die zweite Kommunikationsschnittstelle und damit an den zweiten Steuerschaltkreis überträgt. Unter einer indirekten Kommunikation beziehungsweise einer indirekten Übertragung oder Übermittlung der Information kann verstanden werden, dass mittels der Kommunikationsschnittstelle zunächst das Signal an eine weitere Stelle, beispielsweise eine externe Recheneinheit, übermittelt wird und von dieser dann an die zweite Kommunikationsschnittstelle und somit den zweiten Steuerungsschaltkreis übermittelt wird.

Insbesondere können die bei der Übermittlung der Information verwendeten Signale zur Übermittlung der Information auch verarbeitet oder verändert werden, die Information bleibt dabei jedoch enthalten.

Die drahtlose Übermittlung der Information von dem ersten Steuerungsschaltkreis beziehungsweise der ersten Kommunikationsschnittstelle an den zweiten Steuerungsschaltkreis beziehungsweise die zweite Kommunikationsschnittstelle des kann beispielsweise gemäß einem GSM-Standard, einem auf dem GSM-Standard basierenden Standard, Edge, UMTS, HSDPA, LTE oder einem sonstigen Mobilfunkstandard realisiert sein. Die Übermittlung kann auch beispielsweise basierend auf LTE-M, LTE-CAT-M1 oder einem sonstigen Standard erfolgen. Die Übermittlung kann auch gemäß einem Schmalband-Internet-der-Dinge-Standard, NB-IOT (Englisch: "Narrow Band Internet of Things"), oder einem sonstigen Niedrigenergie-Weitverkehr-Netzwerk, LPWAN (Englisch: "Low Power White Area Network") erfolgen. Die Übermittlung kann auch gemäß einem sonstigen Funkstandard erfolgen, etwa gemäß Bluetooth oder WLAN.

Das Akkupack ist, allein oder in manchen Ausführungsformen zusammen mit einem oder mehreren weiteren Akkupacks, dazu ausgelegt und eingerichtet ist, den elektrischen Verbraucher, insbesondere den Elektromotor, des Elektrowerkzeugs mit Energie zu versorgen. Der erste Steuerschaltkreis kann beispielsweise Teil eines Batteriemanagementsystems des Akkupacks sein.

Das Akkupack kann lösbar, insbesondere zerstörungsfrei lösbar, mit einem Gehäuse des Elektrowerkzeugs verbunden werden, beispielsweise über eine Rastverbindung, eine Steckverbindung und/oder eine Klemmverbindung. Insbesondere kann die mechanische Verbindung des Akkupacks mit dem Gehäuse des Elektrowerkzeugs als formschlüssige und/oder kraftschlüssige Verbindung ausgestaltet sein, ohne dass eine stoffschlüssige Verbindung vorliegt. Mit anderen Worten kann die mechanische Verbindung des Akkupacks mit dem Gehäuse des Elektrowerkzeugs gelöst werden, ohne dass eine stoffschlüssige Verbindung zu lösen ist. Vorzugsweise kann die mechanische Verbindung bestimmungsgemäß manuell ohne den Einsatz sonstiger Werkzeuge gelöst werden. Mit anderen Worten ist das Akkupack als Wechsel-Akkupack, insbesondere System-Akkupack ausgestaltet. Zur elektrischen und mechanischen Verbindung können die Gehäuse des Akkupacks und des Elektrowerkzeugs jeweilige Schnittstellen aufweisen.

Eine elektrische Verbindung der wenigstens einen Akkumulatorzelle mit dem Elektrowerkzeug, insbesondere dem elektrischen Verbraucher, kann beispielsweise über einen oder mehrere lösbare elektrische Kontakte, beispielsweise Klemmkontakte oder Steckkontakte, insbesondere sogenannte Tulpenkontakte oder Schwertkontakte, erfolgen. Beispielsweise können an den Schnittstellen der entsprechenden Gehäuse jeweils zueinander kompatible Stecker oder Buchsen beziehungsweise Aufnahmen oder dergleichen vorgesehen sein, um die elektrische Verbindung des Akkupacks mit dem Elektrowerkzeug zu erzielen.

Durch das erfindungsgemäße Schutzsystem kann anhand der akustischen Warnausgabe der Benutzer des Elektrowerkzeugs, der den Gehörschutz beispielsweise trägt, unmittelbar auf den Wert des Betriebsparameters, beispielsweise im Verhältnis zu einem oder mehreren Grenzwerten, hingewiesen werden oder entsprechend informiert beziehungsweise gewarnt werden.

Die Erfindung nutzt dabei mit Vorteil einen spezifischen akustischen Informationskanal über den Gehörschutz, insbesondere in einen Innenbereich einer Ohrschale des Gehörschutzes. Trägt der Benutzer den Gehörschutz kann er daher die akustische Warnausgabe wahrnehmen, da Eigengeräusche des Elektrowerkzeugs oder Geräusche durch die Wechselwirkung mit einem Werkstück durch den Gehörschutz ausgeblendet oder jedenfalls reduziert sind.

Mit Vorteil kann die akustische Warnmeldung dynamisch erzeugt werden, beispielsweise durch eine Veränderung eines Warntons, einer Tonhöhe des Warntons, eine Frequenz akustischer Impulse des Warnsignals und so weiter, insbesondere abhängig von dem Wert des Betriebsparameters. Dem Benutzer kann auf diese Weise mit einem einfachen akustischen Kommunikationskanal eine relativ hohe Informationsdichte zur Verfügung gestellt werden, die dieser einfach und unmittelbar erfassen und gegebenenfalls darauf reagieren kann.

Dabei ist es besonders vorteilhaft, die Warnausgabe durch den Gehörschutz, beispielsweise in den Innenbereich der, zu erzeugen, da die Warnausgabe aufgrund der durch den Gehörschutz reduzierten Umgebungsgeräusche besonders gut und zuverlässig wahrgenommen werden kann.

Durch die akustische Warnausgabe kann im Idealfall auf eine Abschaltung des Elektrowerkzeugs verzichtet werden, wenn sich der Wert des Betriebsparameters einem kritischen Wert nähert, da dementsprechend frühzeitig und vorzugsweise dynamisch auf die Situation und den Wert des Betriebsparameters hingewiesen werden kann. Mit anderen Worten realisiert die Erfindung eine proaktive Strategie zur Vermeidung kritischer Zustände, im Gegensatz zu einer reaktiven Strategie etwa durch konsequentes Abschalten der Stromversorgung oder Begrenzen der Stromversorgung. Im Vergleich zu einer temperaturabhängigen Abschaltung kann eine erzwungene Abkühlpause des elektrischen Verbrauchers vermieden werden.

Zusätzlich zu der erfindungsgemäßen Ansteuerung der Beleuchtungsvorrichtung abhängig von der übermittelten Information, kann in manchen Ausführungsformen dennoch eine stromabhängige und/oder temperaturabhängige Abschaltung des elektrischen Verbrauchers vorgesehen sein, beispielsweise wenn der Benutzer auf die Ansteuerung der Beleuchtungsvorrichtung nicht oder nicht ausreichend reagiert.

Somit kann nicht nur der Benutzerkomfort bei der Benutzung des Elektrowerkzeugs verbessert werden, sondern auch Qualitätseinbußen bei den Arbeitsergebnissen können vermieden werden, da das Werkzeug möglichst nicht zwischendurch gestoppt wird und wieder anlaufen muss, was zu nicht optimalen Übergängen auf der Werkstückoberfläche und so weiter führen kann, je nach Art des Elektrowerkzeugs.

Zudem wird auch der elektrische Verbraucher zuverlässig geschützt, da das Erreichen des kritischen Zustands möglichst vermieden werden kann. Insbesondere kann eine Überlastung des Elektromotors durch zu hohen Widerstand aufgrund zu hohen Vorschubs durch den Benutzer vermieden werden ebenso wie eine Überlastung der Akkumulatorzellen im Falle eines akkubetriebenen Elektrowerkzeugs und/oder der Elektronik des Akkupacks beziehungsweise des Elektrowerkzeugs.

Bei dem Elektrowerkzeug, das auch ein Gartenwerkzeug sein kann, handelt es sich beispielsweise um ein Elektrowerkzeug zum Sägen, zum Trennen oder zum Bohren. Beispielsweise kann es sich also um eine Säge handeln, etwa eine Stichsäge, eine Kappsäge, eine Tischkreissäge, eine Handkreissäge, eine Kettensäge, einen Hochentaster, eine Bandsäge und so weiter handeln. Es kann sich auch um eine Fräse, einen Winkelschleifer, einen Steintrenner, einen Fliesenschneider, einen Hobel, einen Dickenhobel, eine Drechselbank und so weiter handeln. Darüber hinaus kann es sich um eine Bohrmaschine, eine Schlagbohrmaschine, einen Bohrhammer und so weiter handeln. Diese Auflistung ist lediglich beispielhaft und nicht erschöpfend zu verstehen und beschränkt die Anwendbarkeit der Erfindung nicht grundsätzlich.

Besonders bei Elektrowerkzeugen zum Trennen, Schleifen oder Bohren ist jedoch üblicherweise ein wenigstens teilweise manueller Vorschub durch einen Benutzer zu leisten. Mit anderen Worten hat der Benutzer gegebenenfalls unmittelbaren Einfluss auf die Belastung des Elektrowerkzeugs, insbesondere eines elektrischen Verbrauchers, beispielsweise eines Elektromotors, durch die Beeinflussung des Vorschubs. Im Extremfall kann der Benutzer durch entsprechendes Einwirken auf das Elektrowerkzeug beispielsweise sogar einen Stillstand des Elektromotors provozieren. Besonders bei solchen Elektrowerkzeugen kann die Erfindung daher vorteilhaft eingesetzt werden.

Gemäß zumindest einer Ausführungsform des Schutzsystems ist der erste Steuerschaltkreis dazu eingerichtet, die Information über die erste Kommunikationsschnittstelle und die zweite Kommunikationsschnittstelle direkt an den zweiten Steuerschaltkreis zu übermitteln.

In alternativen Ausführungsformen weist das Schutzsystem eine externe Recheneinheit auf und der erste Steuerschaltkreis ist dazu eingerichtet, die Information über die erste Kommunikationsschnittstelle an die erste Recheneinheit zu übermitteln und die externe Recheneinheit ist dazu eingerichtet, die Information über die zweite Kommunikationsschnittstelle an den zweiten Steuerschaltkreis zu übermitteln, insbesondere um die Information indirekt von dem ersten Steuerschaltkreis zu dem zweiten Steuerschaltkreis zu übermitteln.

Die externe Recheneinheit ist weder Teil des Elektrowerkzeugs noch des Akkupacks noch des Gehörschutzes, ist also extern sowohl zum Elektrowerkzeug als auch zum Akkupack und zum Gehörschutz angeordnet. Bei der externen Recheneinheit kann es sich beispielsweise um eine Recheneinheit eines Servercomputersystems oder eines Cloudcomputersystems oder dergleichen handeln.

Gemäß zumindest einer Ausführungsform ist der Gehörschutz als Kapselgehörschutz ausgestaltet ist und eine, insbesondere ohrmuschelumschließende, aufweist, wobei das Audioausgabegerät beispielsweise in der Ohrschale angeordnet ist.

Dadurch lässt sich die akustische Warnausgabe direkt am Ohr des Benutzers ausgeben, sodass dieser sie besonders gut wahrnehmen kann.

Der Gehörschutz weist insbesondere eine weitere Ohrschale auf, die durch einen Kopfbügel mit der Ohrschale verbunden ist. Der zweite Steuerschaltkreis kann in der Ohrschale oder der weiteren Ohrschale oder in beziehungsweise an dem Kopfbügel angeordnet sein.

Gemäß zumindest einer Ausführungsform weist das Akkupack einen Sensor auf, der dazu angeordnet und eingerichtet ist, abhängig von dem Wert des Betriebsparameters ein Sensorsignal zu erzeugen.

Gemäß zumindest einer Ausführungsform ist der erste Steuerschaltkreis dazu eingerichtet ist, ein von dem Wert des Betriebsparameters abhängendes Sensorsignal von dem Elektrogerät zu erhalten, insbesondere von einem Sensor des Elektrogeräts.

Gemäß zumindest einer Ausführungsform entspricht das Sensorsignal einem Betriebsstrom zum Betrieb eines elektrischen Verbrauchers des Elektrowerkzeugs oder einem von wenigstens einer Akkumulatorzelle des Akkupacks an das Elektrogerät abgegebenen Stroms. Der Sensor des Akkupacks oder des Elektrogeräts ist also insbesondere als Stromsensor ausgestaltet.

Der Betriebsstrom oder der abgegebene Strom stellt eine besonders zuverlässige und einfach zu bestimmende Kenngröße für die Belastung des elektrischen Verbrauchers und/oder des Akkupacks, insbesondere der Akkumulatorzellen, dar.

Gemäß zumindest einer Ausführungsform beinhaltet die Information betreffend den Wert des Betriebsparameters den Wert des Betriebsparameters. Der zweite Steuerschaltkreis ist dazu eingerichtet, das Audioausgabegerät abhängig von dem Wert des Betriebsparameters dazu anzusteuern, die akustische Warnausgabe zu erzeugen.

Gemäß zumindest einer Ausführungsform ist der zweite Steuerschaltkreis dazu eingerichtet, den Wert des Betriebsparameters mit wenigstens einem vorgegebenen Grenzwert zu vergleichen und das Audioausgabegerät abhängig von einem Ergebnis des Vergleichs dazu anzusteuern, die akustische Warnausgabe zu erzeugen.

Gemäß zumindest einer Ausführungsform ist der erste Steuerschaltkreis dazu eingerichtet, den Wert des Betriebsparameters mit wenigstens einem vorgegebenen Grenzwert zu vergleichen und die Information betreffend den Wert des Betriebsparameters beinhaltet ein Ergebnis des Vergleichs. Der zweite Steuerschaltkreis ist dazu eingerichtet, das Audioausgabegerät abhängig von dem Ergebnis des Vergleichs dazu anzusteuern, die akustische Warnausgabe zu erzeugen.

Beispielsweise kann ein erster Grenzwert des wenigstens einen Grenzwerts einen zulässigen Betriebsbereich definieren. Beispielsweise kann der zulässige Betriebsbereich einem Wert des Betriebsparameters kleiner oder gleich dem ersten Grenzwert entsprechen.

In diesem Fall kann eine Lautstärke und/oder Wiederholfrequenz und/oder Tonhöhe der akustischen Warnmeldung beispielsweise umso höher sein, je kleiner der Abstand des Werts des Betriebsparameters von dem ersten Grenzwert ist. Die jeweilige Variation der akustischen Warnmeldung kann dabei kontinuierlich oder in diskreten Schritten, insbesondere in zwei oder mehr diskreten Schritten, erfolgen.

Es kann auch ein zweiter Grenzwert vorgesehen sein, der einen unzulässigen Betriebsbereich definiert. Ist der Wert des Betriebsparameters beispielsweise größer oder gleich dem zweiten Grenzwert, so kann der unzulässige Betriebsbereich gegeben sein. In diesem Fall kann die Lautstärke und/oder Wiederholfrequenz und/oder Tonhöhe der akustischen Warnmeldung beispielsweise auch abhängig von einer Differenz zwischen dem zweiten Grenzwert und dem Wert des Betriebsparameters bestimmt werden, wenn der Wert des Betriebsparameters größer ist als der zweite Grenzwert. Beispielsweise kann die Lautstärke und/oder Wiederholfrequenz und/oder Tonhöhe der akustischen Warnmeldung umso höher sein, je weiter sich der Wert des Betriebsparameters von dem zweiten Grenzwert entfernt.

Gemäß zumindest einer Ausführungsform weist der Gehörschutz, beispielsweise der zweite Steuerschaltkreis, eine Speichereinheit auf, die wenigstens eine Ausgabeinformation speichert. Der zweite Steuerschaltkreis ist dazu eingerichtet, das Audioausgabegerät abhängig von der wenigstens einen Ausgabeinformation dazu anzusteuern, die akustische Warnausgabe zu erzeugen.

Die wenigstens eine Ausgabeinformation enthält zwei oder mehr voneinander verschiedene Ausgabeinformationen. Der zweite Steuerschaltkreis ist dazu eingerichtet, eine der zwei oder mehr verschiedenen Ausgabeinformationen abhängig von der übermittelten Information auszuwählen. Der zweite Steuerschaltkreis ist dazu eingerichtet, das Audioausgabegerät anzusteuern, die akustische Warnausgabe gemäß der ausgewählten Ausgabeinformation zu erzeugen.

So kann durch verschiedene Ausgabeinformationen dynamisch auf den Wert des Betriebsparameters reagiert werden.

Gemäß einem weiteren Aspekt der Erfindung wird ein Elektrowerkzeugsystem aufweisend ein Elektrowerkzeug und erfindungsgemäßes Schutzsystem für das Elektrowerkzeug angegeben.

Gemäß zumindest einer Ausführungsform des Elektrowerkzeugsystems ist das Elektrowerkzeug als Elektrowerkzeug zum Trennen, Schleifen oder Bohren und/oder als Gartengerät ausgestaltet.

Gemäß zumindest einer Ausführungsform ist das Elektrowerkzeug derart ausgestaltet, dass beim Betrieb des Elektrowerkzeugs ein wenigstens teilweise manueller Vorschub durch einen Benutzer vorgesehen ist.

Der erste Steuerschaltkreis kann beispielsweise als erste Recheneinheit ausgestaltet sein oder von einer ersten Recheneinheit beinhaltet sein. Der zweite Steuerschaltkreis kann beispielsweise als zweite Recheneinheit ausgestaltet sein oder von einer zweiten Recheneinheit beinhaltet sein.

Unter einer Recheneinheit kann insbesondere ein Datenverarbeitungsgerät verstanden werden, das einen Verarbeitungsschaltkreis enthält. Die Recheneinheit kann also insbesondere Daten zur Durchführung von Rechenoperationen verarbeiten. Darunter fallen gegebenenfalls auch Operationen, um indizierte Zugriffe auf eine Datenstruktur, beispielsweise eine Umsetzungstabelle, LUT (englisch: "lookup table"), durchzuführen.

Die Recheneinheit kann insbesondere einen oder mehrere Computer, einen oder mehrere Mikrocontroller und/oder einen oder mehrere integrierte Schaltkreise enthalten, beispielsweise eine oder mehrere anwendungsspezifische integrierte Schaltungen, ASIC (englisch: "application-specific integrated circuit"), eines oder mehrere feldprogrammierbare Gate-Arrays, FPGA, und/oder eines oder mehrere Einchipsysteme, SoC (englisch: "system on a chip"). Die Recheneinheit kann auch einen oder mehrere Prozessoren, beispielsweise einen oder mehrere Mikroprozessoren, eine oder mehrere zentrale Prozessoreinheiten, CPU (englisch: "central processing unit"), eine oder mehrere Grafikprozessoreinheiten, GPU (englisch: "graphics processing unit") und/oder einen oder mehrere Signalprozessoren, insbesondere einen oder mehrere digitale Signalprozessoren, DSP, enthalten. Die Recheneinheit kann auch einen physischen oder einen virtuellen Verbund von Computern oder sonstigen der genannten Einheiten beinhalten.

In verschiedenen Ausführungsbeispielen beinhaltet die Recheneinheit eine oder mehrere Hardware- und/oder Softwareschnittstellen und/oder eine oder mehrere Speichereinheiten.

Eine Speichereinheit kann als flüchtiger Datenspeicher, beispielsweise als dynamischer Speicher mit wahlfreiem Zugriff, DRAM (englisch: "dynamic random access memory") oder statischer Speicher mit wahlfreiem Zugriff, SRAM (englisch: "static random access memory"), oder als nicht-flüchtiger Datenspeicher, beispielsweise als Festwertspeicher, ROM (englisch: "read-only memory"), als programmierbarer Festwertspeicher, PROM (englisch: "programmable read-only memory"), als löschbarer programmierbarer Festwertspeicher, EPROM (englisch: "erasable programmable read-only memory"), als elektrisch löschbarer programmierbarer Festwertspeicher, EEPROM (englisch: "electrically erasable programmable read-only memory"), als Flash-Speicher oder Flash-EEPROM, als ferroelektrischer Speicher mit wahlfreiem Zugriff, FRAM (englisch: "ferroelectric random access memory"), als magnetoresistiver Speicher mit wahlfreiem Zugriff, MRAM (englisch: "magnetoresistive random access memory") oder als Phasenänderungsspeicher mit wahlfreiem Zugriff, PCRAM (englisch: "phase-change random access memory"), ausgestaltet sein.

Ist im Rahmen der vorliegenden Offenbarung die Rede davon, dass eine Komponente des erfindungsgemäßen Schutzsystems oder des erfindungsgemäßen Elektrowerkzeugsystems, insbesondere der erste Steuerschaltkreis, der zweite erste Steuerschaltkreis, die externe Recheneinheit oder eine sonstige Recheneinheit des Schutzsystems oder des Elektrowerkzeugsystems dazu eingerichtet, ausgebildet, ausgelegt, oder dergleichen ist, eine bestimmte Funktion auszuführen oder zu realisieren, eine bestimmte Wirkung zu erzielen oder einem bestimmten Zweck zu dienen, so kann dies derart verstanden werden, dass die Komponente, über die prinzipielle oder theoretische Verwendbarkeit oder Eignung der Komponente für diese Funktion, Wirkung oder diesen Zweck hinaus, durch eine entsprechende Anpassung, Programmierung, physische Ausgestaltung und so weiter konkret und tatsächlich dazu in der Lage ist, die Funktion auszuführen oder zu realisieren, die Wirkung zu erzielen oder dem Zweck zu dienen.

Weitere Merkmale der Erfindung ergeben sich aus den Ansprüchen, den Figuren und der Figurenbeschreibung. Die vorstehend in der Beschreibung genannten Merkmale und Merkmalskombinationen sowie die nachfolgend in der Figurenbeschreibung genannten und/oder in den Figuren gezeigten Merkmale und Merkmalskombinationen können nicht nur in der jeweils angegebenen Kombination, sondern auch in anderen Kombinationen von der Erfindung umfasst sein. Es können insbesondere auch Ausführungen und Merkmalskombinationen von der Erfindung umfasst sein, die nicht alle Merkmale eines ursprünglich formulierten Anspruchs aufweisen. Es können darüber hinaus Ausführungen und Merkmalskombinationen von der Erfindung umfasst, die über die in den Rückbezügen der Ansprüche dargelegten Merkmalskombinationen hinausgehen oder von diesen abweichen.

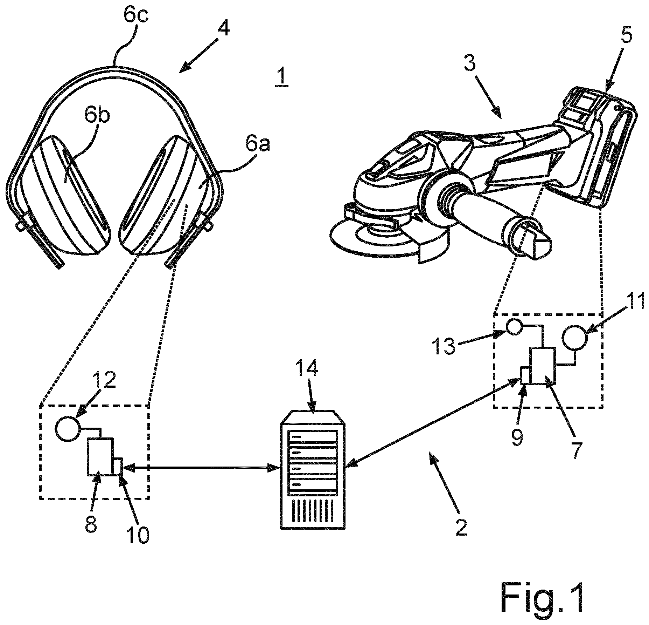

Die Erfindung wird im Folgenden anhand konkreter Ausführungsbeispiele und zugehöriger schematischer Zeichnungen näher erläutert. Dabei zeigt
- Fig. 1: eine schematische Darstellung einer beispielhaften Ausführungsform eines erfindungsgemäßen Elektrowerkzeugsystems.

In der Fig. 1 ist schematisch eine beispielhafte Ausführungsform eines erfindungsgemäßen Elektrowerkzeugsystems 1 gezeigt, das ein Elektrowerkzeug 3, rein beispielhaft als Winkelschleifer dargestellt, und eine beispielhafte Ausführungsform eines erfindungsgemäßen Schutzsystems 2 für das Elektrowerkzeug 3 auf.

Das Schutzsystem 2 weist ein Akkupack 5 mit Akkumulatorzellen 11 auf, das mit dem Elektrowerkzeug 3 zur Versorgung eines elektrischen Verbrauchers, insbesondere Elektromotors (nicht gezeigt) des Elektrowerkzeugs 3 mit elektrischer Energie verbindbar ist, sowie einen Gehörschutz 4, beispielhaft als Kapselgehörschutz mit zwei Ohrschalen 6a, 6b und einem die Ohrschalen 6a, 6b verbindenden Kopfbügel 6c dargestellt.

Das Schutzsystem 2 weist einen Sensor 13 des Elektrowerkzeugs 3 oder des Akkupacks auf, der angeordnet und eingerichtet ist, einen Wert eines Betriebsparameters des Elektrowerkzeugs 3 zu bestimmen oder zu messen und abhängig von dem Wert des Betriebsparameters des Elektrowerkzeugs 3 ein Sensorsignal zu erzeugen.

Der Sensor 13 kann beispielsweise als Stromsensor ausgestaltet sein und der Betriebsparameter kann einem Strom von dem Akkupack 5 oder den Akkumulatorzellen 11 zu dem Elektromotor entsprechen. Je nach Ausgestaltung des Elektrowerkzeugs 3 sind auch andere Betriebsparameter und entsprechende Sensoren 13 möglich.

Das Akkupack 5 weist einen ersten Steuerschaltkreis 7 auf, der dazu eingerichtet ist, abhängig von dem Sensorsignal eine Information betreffend den Wert des Betriebsparameters an einen zweiten Steuerschaltkreis 8 des Gehörschutzes 4 zu übermitteln.

Der Gehörschutz 4 weist ein Audioausgabegerät 12 auf, beispielsweise einen Lautsprecher, und der zweite Steuerschaltkreis 8 ist dazu eingerichtet, das Audioausgabegerät 12 abhängig von der übermittelten Information dazu anzusteuern, eine akustische Warnausgabe zu erzeugen.

Der zweite Steuerschaltkreis 8 und/oder das Audioausgabegerät 12 können beispielsweise in einer der Ohrschalen 6a, 6b angeordnet sein, sodass das Audioausgabegerät 12 die akustische Warnausgabe in Richtung eines Ohrs des Benutzers abgeben kann, wenn dieser den Gehörschutz bestimmungsgemäß trägt.

Der erste Steuerschaltkreis 7 ist dazu eingerichtet, abhängig von dem Sensorsignal eine Information betreffend den Wert des Betriebsparameters direkt an den zweiten Steuerschaltkreis 8 zu übermitteln, insbesondere über eine erste Kommunikationsschnittstelle 9 des Akkupacks 5, beispielsweise des ersten Steuerschaltkreises 7, und eine zweite Kommunikationsschnittstelle 10 des Gehörschutzes 4. Alternativ kann der erste Steuerschaltkreis 7 abhängig von dem Sensorsignal die Information betreffend den Wert des Betriebsparameters über die erste Kommunikationsschnittstelle 9 an eine externe Recheneinheit 14, insbesondere einen Cloudserver oder dergleichen, des Schutzsystems 2 übermitteln und die externe Recheneinheit 14 kann die Information betreffend den Wert des Betriebsparameters an die zweite Kommunikationsschnittstelle 10 und damit an den zweiten Steuerschaltkreis 8 übermitteln.

### BEZUGSZEICHENLISTE:

- 1: Elektrowerkzeugsystem
- 2: Schutzsystem
- 3: Elektrowerkzeug
- 4: Gehörschutz
- 5: Akkupack
- 6a, 6b: Ohrschalen
- 6c: Kopfbügel
- 7, 8: Steuerschaltkreise
- 9, 10: Kommunikationsschnittstellen
- 11: Akkumulatorzellen
- 12: Audioausgabegerät
- 13: Sensor
- 14: externe Recheneinheit

## Patentansprüche

1. Schutzsystem (2) für ein Elektrowerkzeug (3), das Schutzsystem (2) aufweisend ein Akkupack (5), das mit dem Elektrowerkzeug (3) zur Versorgung eines elektrischen Verbrauchers des Elektrowerkzeugs (3) mit elektrischer Energie verbindbar ist, sowie einen Gehörschutz (4) für einen Benutzer des Elektrowerkzeugs (3), wobei
- das Akkupack (5) einen ersten Steuerschaltkreis (7) aufweist, der dazu eingerichtet ist, eine Information betreffend einen Wert eines Betriebsparameters des Elektrowerkzeugs (3) direkt oder indirekt an einen zweiten Steuerschaltkreis (8) des Gehörschutzes (4) zu übermitteln; und
- der Gehörschutz (4) ein Audioausgabegerät (12) aufweist und der zweite Steuerschaltkreis (8) dazu eingerichtet ist, das Audioausgabegerät (12) abhängig von der übermittelten Information dazu anzusteuern, eine akustische Warnausgabe zu erzeugen.

2. Schutzsystem (2) nach Anspruch 1, wobei der Gehörschutz (4) als Kapselgehörschutz (4) ausgestaltet ist und eine Ohrschale (6a, 6b) aufweist und das Audioausgabegerät (12) in der Ohrschale (6a, 6b) angeordnet ist.

3. Schutzsystem (2) nach einem der vorhergehenden Ansprüche, wobei der erste Steuerschaltkreis (7) dazu eingerichtet ist, ein von dem Wert des Betriebsparameters abhängendes Sensorsignal von dem Elektrogerät zu erhalten.

4. Schutzsystem (2) nach einem der Ansprüche 1 oder 2, wobei das Akkupack (5) einen Sensor (13) aufweist, der eingerichtet ist, abhängig von dem Wert des Betriebsparameters ein Sensorsignal zu erzeugen.

5. Schutzsystem (2) nach einem der vorhergehenden Ansprüche, das Sensorsignal einem Betriebsstrom zum Betrieb eines elektrischen Verbrauchers des Elektrowerkzeugs (3) oder einem von wenigstens einer Akkumulatorzelle (11) des Akkupacks (5) an das Elektrogerät abgegebenen Stroms entspricht.

6. Schutzsystem (2) nach einem der Ansprüche 3 bis 5, wobei der erste Steuerschaltkreis (7) dazu eingerichtet ist, die Information betreffend den Wert des Betriebsparameters abhängig von dem Sensorsignal an den zweiten Steuerschaltkreis (8) zu übermitteln.

7. Schutzsystem (2) nach einem der vorhergehenden Ansprüche, wobei
- das Akkupack (5) eine mit dem ersten Steuerschaltkreis (7) verbundene erste Kommunikationsschnittstelle aufweist; und
- der Gehörschutz (4) eine mit dem zweiten Steuerschaltkreis (8) verbundene zweite Kommunikationsschnittstelle aufweist.

8. Schutzsystem (2) nach Anspruch 7, wobei
- der erste Steuerschaltkreis (7) dazu eingerichtet ist, die Information über die erste Kommunikationsschnittstelle (9) und die zweite Kommunikationsschnittstelle (10) direkt an den zweiten Steuerschaltkreis (8) zu übermitteln; oder
- das Schutzsystem (2) eine externe Recheneinheit (14) aufweist, der erste Steuerschaltkreis (7) dazu eingerichtet ist, die Information über die erste Kommunikationsschnittstelle (9) an die externe Recheneinheit (14) zu übermitteln, und die externe Recheneinheit (14) dazu eingerichtet ist, die Information über die zweite Kommunikationsschnittstelle (10) an den zweiten Steuerschaltkreis (8) zu übermitteln.

9. Schutzsystem (2) nach einem der vorhergehenden Ansprüche, wobei
- die Information betreffend den Wert des Betriebsparameters den Wert des Betriebsparameters beinhaltet; und
- der zweite Steuerschaltkreis (8) dazu eingerichtet ist, das Audioausgabegerät (12) abhängig von dem Wert des Betriebsparameters dazu anzusteuern, die akustische Warnausgabe zu erzeugen.

10. Schutzsystem (2) nach Anspruch 9, wobei der zweite Steuerschaltkreis (8) dazu eingerichtet ist, den Wert des Betriebsparameters mit wenigstens einem vorgegebenen Grenzwert zu vergleichen und das Audioausgabegerät (12) abhängig von einem Ergebnis des Vergleichs dazu anzusteuern, die akustische Warnausgabe zu erzeugen.

11. Schutzsystem (2) nach einem der Ansprüche 1 bis 8, wobei
- der erste Steuerschaltkreis (7) dazu eingerichtet ist, den Wert des Betriebsparameters mit wenigstens einem vorgegebenen Grenzwert zu vergleichen und die Information betreffend den Wert des Betriebsparameters ein Ergebnis des Vergleichs beinhaltet; und
- der zweite Steuerschaltkreis (8) dazu eingerichtet ist, das Audioausgabegerät (12) abhängig von dem Ergebnis des Vergleichs dazu anzusteuern, die akustische Warnausgabe zu erzeugen.

12. Schutzsystem (2) nach einem der vorhergehenden Ansprüche, wobei
- der Gehörschutz (4) eine Speichereinheit aufweist, die wenigstens eine Ausgabeinformation speichert; und
- der zweite Steuerschaltkreis (8) dazu eingerichtet ist, das Audioausgabegerät (12) abhängig von der wenigstens einen Ausgabeinformation dazu anzusteuern, die akustische Warnausgabe zu erzeugen.

13. Schutzsystem (2) nach Anspruch 12, wobei
- die wenigstens eine Ausgabeinformation zwei oder mehr voneinander verschiedene Ausgabeinformationen enthält; und
- der zweite Steuerschaltkreis (8) dazu eingerichtet ist, eine der zwei oder mehr verschiedenen Ausgabeinformationen abhängig von der übermittelten Information auszuwählen; und
- der zweite Steuerschaltkreis (8) dazu eingerichtet ist, das Audioausgabegerät (12) anzusteuern, die akustische Warnausgabe gemäß der ausgewählten Ausgabeinformation zu erzeugen.

14. Elektrowerkzeugsystem (1) aufweisend ein Elektrowerkzeug (3) und ein Schutzsystem (2) für das Elektrowerkzeug (3) nach einem der vorhergehenden Ansprüche.

15. Elektrowerkzeugsystem (1) nach Anspruch 14, wobei
- das Elektrowerkzeug (3) als Elektrowerkzeug (3) zum Trennen, Schleifen oder Bohren ausgestaltet ist; oder
- das Elektrowerkzeug (3) als Gartengerät ausgestaltet ist; oder
- das Elektrowerkzeug (3) derart ausgestaltet ist, dass beim Betrieb des Elektrowerkzeugs (3) ein wenigstens teilweise manueller Vorschub durch den Benutzer vorgesehen ist.

## Claims

1. Protection system (2) for a power tool (3), the protection system (2) comprising a battery pack (5), which is connectable with the power tool (3) for supplying an electrical load of the power tool (3) with electric energy, as well as a hearing protection (4) for a user of the power tool (3), wherein
- the battery pack (5) comprises a first control circuit (7), which is configured to transmit an information concerning a value of an operating parameter of the power tool (3) directly or indirectly to a second control circuit (8) of the hearing protection (4); and
- the hearing protection (4) comprises an audio output device (12) and the second control circuit (8) is configured to control the audio output device (12) depending on the transmitted information to generate an acoustic warning output.

2. Protection system (2) according to claim 1, wherein the hearing protection (4) is designed as capsule hearing protection (4) and comprises an ear cup (6a, 6b) and the audio output device (12) is arranged in the ear cup (6a, 6b).

3. Protection system (2) according to any one of the preceding claims, wherein the first control circuit (7) is configured to receive a sensor signal depending on the value of the operating parameter from the electric appliance.

4. Protection system (2) according to any one of claims 1 or 2, wherein the battery pack (5) comprises a sensor (13), which is configured to generate a sensor signal depending on the value of the operating parameter.

5. Protection system (2) according to any one of the preceding claims, wherein the sensor signal corresponds to an operating current for operating an electrical load of the power tool (3) or to a current output by one of at least one accumulator cell (11) of the battery pack (5) to the electric appliance.

6. Protection system (2) according to any one of claims 3 to 5, wherein the first control circuit (7) is configured to transmit the information concerning the value of the operating parameter depending on the sensor signal to the second control circuit (8).

7. Protection system (2) according to any one of the preceding claims, wherein
- the battery pack (5) comprises a first communication interface connected to the first control circuit (7); and
- the hearing protection (4) comprises a second communication interface connected to the second control circuit (8).

8. Protection system (2) according to claim 7, wherein
- the first control circuit (7) is configured to transmit the information via the communication interface (9) and the second communication interface (10) directly to the second control circuit (8); or
- the protection system (2) comprises an external computing unit (14), the first control circuit (7) is configured to transmit the information via the first communication interface (9) to the external computing unit (14), and the external computing unit (14) is configured to transmit the information via the second communication interface (10) to the second control circuit (8).

9. Protection system (2) according to any one of the preceding claims, wherein
- the information concerning the value of the operating parameter comprises the value of the operating parameter; and
- the second control circuit (8) is configured to control the audio output device (12) depending on the value of the operating parameter to generate the acoustic warning output.

10. Protection system (2) according to claim 9, wherein the second control circuit (8) is configured to compare the value of the operating parameter with at least one predetermined limiting value and to control the audio output device (12) depending on a result of the comparison to generate the acoustic warning output.

11. Protection system (2) according to any one of claims 1 to 8, wherein
- the first control circuit (7) is configured to compare the value of the operating parameter with the at least one predetermined limiting value and the information concerning the value of the operating parameter comprises a result of the comparison; and
- the second control circuit (8) is configured to control the audio output device (12) depending on the result of the comparison to generate the acoustic warning output.

12. Protection system (2) according to any one of the preceding claims, wherein
- the hearing protection (4) comprises a memory unit, which stores at least one output information; and
- the second control circuit (8) is configured to control the audio output device (12) depending on the at least one output information to generate the acoustic warning output.

13. Protection system (2) according to claim 12, wherein
- the at least one output information comprises two or more different pieces of output information; und
- the second control circuit (8) is configured to select one of the two or more different pieces of output information depending on the transmitted information; and
- the second control circuit (8) is configured to control the audio output device (12) to generate the acoustic warning output according to the selected output information.

14. Power tool system (1) comprising a power tool (3) and a protection system (2) for the power tool (3) according to any one of the preceding claims.

15. Power tool system (1) according to claim 14, wherein
- the power tool (3) is designed as power tool (3) for cutting, grinding or drilling; or
- the power tool (3) is designed as gardening tool; or
- the power tool (3) is designed such that during operation of the power tool (3) an at least in part manual feed by the user is provided.

## Revendications

1. Système de protection (2) destiné à un outil électrique (3), le système de protection (2) comportant une batterie (5) qui peut être reliée à l'outil électrique (3) afin d'alimenter un consommateur électrique de l'outil électrique (3) en énergie électrique, et une protection auditive (4) destinée à un utilisateur de l'outil électrique (3),
- la batterie (5) comportant un premier circuit de commande (7) qui est conçu pour transmettre une information relative à une valeur d'un paramètre de fonctionnement de l'outil électrique (3) directement ou indirectement à un deuxième circuit de commande (8) de la protection auditive (4) ; et
- la protection auditive (4) comportant un dispositif de sortie audio (12) et le deuxième circuit de commande (8) étant conçu pour commander le dispositif de sortie audio (12) en fonction de l'information transmise afin de générer une sortie d'avertissement acoustique.

2. Système de protection (2) selon la revendication 1, la protection auditive (4) étant conçue comme un serre-tête (4) et comportant une coque d'oreille (6a, 6b) et le dispositif de sortie audio (12) étant disposé dans la coque d'oreille (6a, 6b).

3. Système de protection (2) selon l'une des revendications précédentes, le premier circuit de commande (7) étant conçu pour recevoir du dispositif électrique un signal de capteur qui dépend de la valeur du paramètre de fonctionnement.

4. Système de protection (2) selon l'une des revendications 1 ou 2, la batterie (5) comportant un capteur (13) qui est conçu pour générer un signal de capteur en fonction de la valeur du paramètre de fonctionnement.

5. Système de protection (2) selon l'une des revendications précédentes, le signal de capteur correspondant à un courant de fonctionnement destiné à faire fonctionner un consommateur électrique de l'outil électrique (3) ou à un courant délivré au dispositif électrique par au moins une cellule d'accumulateur (11) de la batterie (5).

6. Système de protection (2) selon l'une des revendications 3 à 5, le premier circuit de commande (7) étant conçu pour transmettre l'information relative à la valeur du paramètre de fonctionnement au deuxième circuit de commande (8) en fonction du signal de capteur.

7. Système de protection (2) selon l'une des revendications précédentes,
- la batterie (5) comportant une première interface de communication reliée au premier circuit de commande (7) ; et
- la protection auditive (4) comportant une deuxième interface de communication reliée au deuxième circuit de commande (8).

8. Système de protection (2) selon la revendication 7,
- le premier circuit de commande (7) étant conçu pour transmettre l'information directement au deuxième circuit de commande (8) par le biais de la première interface de communication (9) et de la deuxième interface de communication (10) ; ou
- le système de protection (2) comportant une unité informatique externe (14), le premier circuit de commande (7) étant conçu pour transmettre l'information à l'unité informatique externe (14) par le biais de la première interface de communication (9), et l'unité informatique externe (14) étant conçue pour transmettre l'information au deuxième circuit de commande (8) par le biais de la deuxième interface de communication (10) .

9. Système de protection (2) selon l'une des revendications précédentes,
- l'information relative à la valeur du paramètre de fonctionnement contenant la valeur du paramètre de fonctionnement ; et
- le deuxième circuit de commande (8) étant conçu pour commander le dispositif de sortie audio (12) en fonction de la valeur du paramètre de fonctionnement afin de générer la sortie d'avertissement acoustique.

10. Système de protection (2) selon la revendication 9, le deuxième circuit de commande (8) étant conçu pour comparer la valeur du paramètre de fonctionnement à au moins une valeur limite spécifiée et pour commander le dispositif de sortie audio (12) en fonction du résultat de la comparaison afin de générer la sortie d'avertissement acoustique.

11. Système de protection (2) selon l'une des revendications 1 à 8,
- le premier circuit de commande (7) étant conçu pour comparer la valeur du paramètre de fonctionnement à au moins une valeur limite spécifiée et l'information relative à la valeur du paramètre de fonctionnement contenant le résultat de la comparaison ; et
- le deuxième circuit de commande (8) étant conçu pour commander le dispositif de sortie audio (12) en fonction du résultat de la comparaison afin de générer la sortie d'avertissement acoustique.

12. Système de protection (2) selon l'une des revendications précédentes,
- la protection auditive (4) comportant une unité de mémorisation qui mémorise au moins une information de sortie ; et
- le deuxième circuit de commande (8) étant conçu pour commander le dispositif de sortie audio (12) en fonction de l'au moins une information de sortie afin de générer la sortie d'avertissement acoustique.

13. Système de protection (2) selon la revendication 12,
- l'au moins une information de sortie contenant deux informations de sortie différentes ou plus ; et
- le deuxième circuit de commande (8) étant conçu pour sélectionner l'une des deux informations de sortie différentes ou plus en fonction de l'information transmise ; et
- le deuxième circuit de commande (8) étant conçu pour commander le dispositif de sortie audio (12) afin de générer la sortie d'avertissement acoustique en fonction de l'information de sortie sélectionnée.

14. Système d'outil électrique (1) comprenant un outil électrique (3) et un système de protection (2) destiné à l'outil électrique (3) selon l'une des revendications précédentes.

15. Système d'outil électrique (1) selon la revendication 14,
- l'outil électrique (3) étant conçu comme un outil électrique (3) de tronçonnage, de meulage ou de perçage ; ou
- l'outil électrique (3) étant conçu comme un dispositif de jardinage ; ou
- l'outil électrique (3) étant conçu de manière à assurer un avancement au moins partiellement manuel par l'utilisateur pendant le fonctionnement de l'outil électrique (3).
